# FASCICULE DE BREVET EUROPEEN

(11) **EP 4 337 637 B1**
(45) Date de publication et mention de la délivrance du brevet: **25.02.2026**
(21) Numéro de dépôt: 22727376.0
(22) Date de dépôt: 10.05.2022
(51) Int. Cl.: C07C 319/08, C07C 319/28, C07C 319/24, C07C 321/04, C07C 321/14

(54) **PROCÉDÉ DE CO-PRODUCTION D'ALKYLMERCAPTAN ET DE DIALKYLDISULFURE À PARTIR D'ALCOOL**
VERFAHREN ZUR GLEICHZEITIGEN HERSTELLUNG VON ALKYLMERCAPTAN UND DIALKYLDISULFID AUS ALKOHOL
PROCESS FOR THE CO-PRODUCTION OF ALKYL MERCAPTAN AND DIALKYL DISULFIDE FROM ALCOHOL

(30) Priorité: 11.05.2021 FR 2104977
(43) Date de publication de la demande: 20.03.2024
(73) Titulaire: ARKEMA FRANCE, 92800 Puteaux (FR)
(72) Inventeur: FREMY, Georges, 64170 Lacq (FR); RAYMOND, Jean-Michel, 40300 Cauneille (FR); LAMANT, Eric, 64170 Lacq (FR)
(74) Mandataire: Arkema Patent
(86) Numéro de dépôt international: PCT/FR2022/050888
(87) Numéro de publication internationale: WO 2022/238650

(56) Documents cités:
- EP-A2- 0 171 092
- WO-A1-2016/001553
- WO-A1-2016/001554
- FR-A1- 2 935 142

## Description

La présente invention concerne un procédé de co-production d'alkylmercaptan et de dialkyldisulfure à partir d'alcool.

Les mercaptans présentent un grand intérêt industriel et sont aujourd'hui très largement utilisés par les industries chimiques, notamment comme matières premières pour la synthèse de molécules organiques plus complexes. Par exemple, le méthylmercaptan (noté CH₃SH ou MeSH ci-après) est utilisé comme matière première dans la synthèse de la méthionine, acide aminé essentiel pour l'alimentation animale. Le méthylmercaptan est également utilisé dans la synthèse de dialkyldisulfures, en particulier dans la synthèse du disulfure de diméthyle (noté DMDS ci-après).

Les dialkyldisulfures, et notamment le disulfure de diméthyle, présentent eux aussi un grand intérêt industriel et sont très largement utilisés dans l'industrie. Par exemple, et de manière non limitative, ils sont utilisés en tant qu'additif de sulfuration de catalyseurs, notamment dans l'hydrotraitement de coupes pétrolières, en tant qu'additif anti-coke et anti-CO dans les charges pétrolières soumises au vapocraquage pour la production d'éthylène, ou encore comme agent de fumigation des sols dans l'agriculture.

Par rapport à d'autres produits utilisés dans ces applications, comme par exemple les di-tertio-alkylpolysulfures, les disulfures organiques, et en particulier le DMDS, présentent de nombreux avantages. Par exemple, le DMDS présente une teneur en soufre élevée (68 %) et des produits de dégradation non cokants (CH₄, H₂S). De plus, dans ces applications, le DMDS conduit à des performances généralement supérieures aux autres produits commerciaux et habituellement utilisés, par exemple les di-tertio-alkylpolysulfures.

Aujourd'hui, il est connu de produire le méthylmercaptan selon différentes voies de synthèse.

Le méthylmercaptan peut être produit à partir de méthanol (CH₃OH) et de sulfure d'hydrogène (H₂S) selon la réaction (1) suivante :

CH₃OH + H₂S → CH₃SH + H₂O (1)

Il est également possible de préparer le méthylmercaptan à partir de monoxyde de carbone (CO) selon la réaction (2) suivante :

CO + 2H₂ + H₂S → CH₃SH + H₂O (2)

D'autres procédés encore sont décrits dans la littérature et combinent différentes réactions telles que :
- formation de CS₂ et d'H₂ à partir de méthane et de soufre, selon la réaction (3) :

   CH₄ + H₂S + S → CS₂ + 3 H₂ (3)
- hydrogénation du CS₂, avec l'hydrogène formé ci-dessus, selon la réaction (4) :

   CS₂ + 3 H₂ → CH₃SH + H₂S (4)

La synthèse du diméthyldisulfure se fait classiquement par oxydation avec du soufre selon la réaction (5) suivante :

2 CH₃SH + S → CH₃SSCH₃ + H₂S (5).

Cette oxydation des alkylmercaptans par le soufre, catalysée par des agents basiques organiques ou inorganiques, homogènes ou hétérogènes, en discontinu ou en continu, s'accompagne d'une formation de sulfure d'hydrogène ainsi que de dialkylpolysulfures, notés RSxR de rang en soufre x supérieur à 2 (par exemple polysulfures de diméthyle CH₃SxCH₃ dans le cas de la synthèse de DMDS). Par ailleurs, cette étape de synthèse demande généralement un excès important de méthylmercaptan.

FR2935142A1 et EP0171092A2 divulguent des procédés pour préparer méthylmercaptan et son transformation en diméthyldisulfure.

Or, au regard des considérations écologiques actuelles, il existe aujourd'hui un réel besoin pour un procédé de synthèse d'alkylmercaptans et de dialkyldisulfures plus respectueux de l'environnement, tout en conservant des rendements élevés.

### Brève description de l'invention

Ainsi, la présente invention a pour objet un procédé de co-production d'alkylmercaptan et de dialkyldisulfure comprenant les étapes successives suivantes :
a) réaction d'un alcool en C₁-C₄ en présence de sulfure d'hydrogène (H₂S) pour former un flux (M) comprenant un alkylmercaptan, de l'eau, et éventuellement du sulfure d'hydrogène n'ayant pas réagi,
b) purification du flux (M) pour obtenir un flux (N) enrichi en alkylmercaptan,
c) récupération d'une première partie du flux (N) comportant de l'alkylmercaptan purifié à l'étape b),
d) oxydation par du soufre de la seconde partie du flux (N) d'alkylmercaptan, pour former un flux (O) comprenant un dialkyldisulfure, du sulfure d'hydrogène, et éventuellement de l'alkylmercaptan n'ayant pas réagi,
e) purification du flux (O) pour séparer d'une part le dialkyldisulfure enrichi et d'autre part le sulfure d'hydrogène et éventuellement l'alkylmercaptan n'ayant pas réagi à l'étape d),
f) recyclage du sulfure d'hydrogène et éventuellement de l'alkylmercaptan isolés lors de l'étape
e) vers le flux (M) issu de l'étape a),
g) récupération du dialkyldisulfure isolé à l'étape e).

Ce procédé permet la synthèse en continu d'alkylmercaptan et de dialkyldisulfure. Cette co-production de produits permet de diminuer le coût énergétique de la synthèse. Cette économie énergétique est un premier avantage écologique.

Elle permet également de moduler la production de chaque produit en fonction de la demande. Par exemple, la synthèse d'alkylmercaptan peut être privilégiée par rapport à celle du dialkyldisulfure. Cette flexibilité dans le procédé est aussi un avantage. Il est également possible selon les besoins de ne produire que l'alkylmercaptan, c'est-à-dire arrêter le procédé à l'étape c). De même, en cas de besoin, la totalité du flux (N) peut être engagée dans l'étape d) d'oxydation. Cette flexibilité du procédé présente un avantage considérable. Il permet d'adapter la production des produits selon les besoins, sur une seule et unique installation.

Ensuite, cette co-production permet de recycler les impuretés du produit final. L'alkylmercaptan n'ayant pas réagi lors de la réaction d'oxydation par le soufre et le sulfure d'hydrogène généré au cours de cette étape d'oxydation sont recyclés au niveau de la synthèse d'alkylmercaptan. Habituellement, ces impuretés sont incinérées, conduisant à la formation d'oxydes de soufre (SO₂), potentiellement responsables de pluies acides. A l'heure actuelle, ces rejets ne sont plus tolérés. Or, le recyclage de la totalité de ces impuretés légères permet d'éviter leur incinération. L'étape f) de recyclage selon l'invention permet ainsi un recyclage du sulfure d'hydrogène sur une installation fermée. Le sulfure d'hydrogène étant un gaz toxique, le recyclage fermé permet de limiter la manipulation de ce gaz, et de ce fait, de limiter les accidents.

Il serait également possible de séparer le sulfure d'hydrogène de l'alkylmercaptan pour valoriser ces impuretés. Or, cette séparation est très difficile, elle nécessite une distillation équipée d'une très haute colonne. Cette séparation est, de ce fait, très consommatrice d'énergie. Ainsi, le recyclage de ces deux impuretés, au sein d'un même flux (c'est-à-dire non séparées) pour être intégré dans une étape de purification déjà existante du procédé de synthèse, est une solution simple et énergétiquement très avantageuse ; d'autant plus, que l'étape d'oxydation au soufre nécessite généralement un très large excès d'alkylmercaptan.

Ce recyclage est intégré au niveau d'une étape de purification indispensable au procédé de synthèse. Ce recyclage est ainsi simple à mettre en œuvre et peu couteux en énergie. Il ne nécessite pas d'étape supplémentaire dans le procédé de synthèse.

Enfin, ces impuretés, qui sont des composés de la première étape de synthèse : réactif pour le sulfure d'hydrogène et produit pour l'alkylmercaptan, enrichissent cette première étape du procédé, conduisant à une diminution de la consommation de matières premières.

Sur le plan réactionnel, le procédé revendiqué couvre les deux réactions suivantes : ROH + H₂S → RSH + H₂O

2RSH + S → RSSR + H₂S

Ces réactions peuvent être simplifiées de la façon suivante, lorsque le sulfure d'hydrogène est recyclé dans la première étape :

2ROH + H₂S + S → RSSR + 2H₂O

### Brève description de la figure

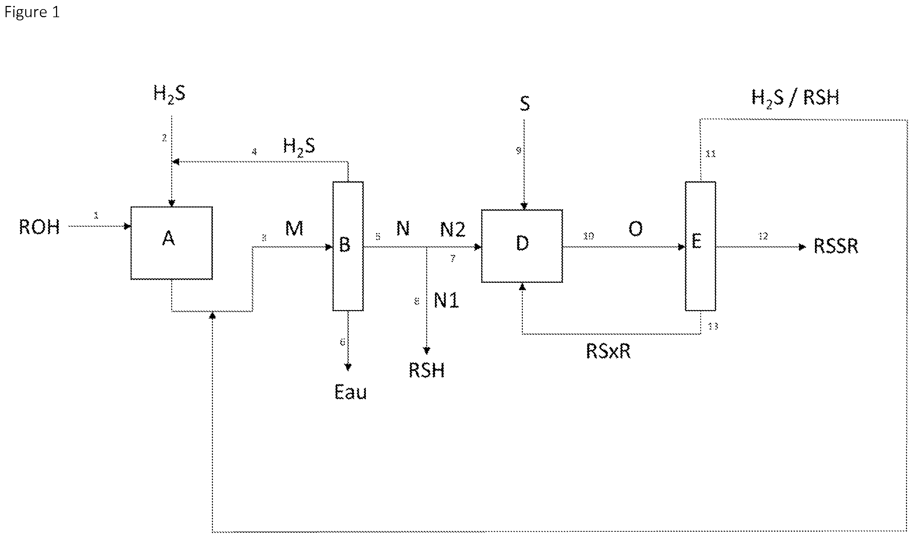

La figure 1 est un schéma du dispositif mettant en œuvre le procédé revendiqué.

### Description détaillée de l'invention

D'autres caractéristiques, aspects, objets et avantages de la présente invention apparaîtront encore plus clairement à la lecture de la description qui suit.

Il est précisé que les expressions « de ...à ... » et « compris entre ... et .... » utilisées dans la présente description doivent s'entendre comme incluant chacune des bornes mentionnées.

Le procédé selon l'invention comprend les sept étapes consécutives précitées : étapes a) à g). Ce procédé peut comporter des étapes de purification intermédiaires.

### Etape a) - Réaction

Lors de l'étape a), on fait réagir un alcool en C₁-C₄ avec du sulfure d'hydrogène pour former un flux (M) comprenant un alkylmercaptan en C₁-C₄, de l'eau, éventuellement du sulfure d'hydrogène n'ayant pas réagi et éventuellement des sous-produits soufrés.

L'alcool en C₁-C₄ selon l'invention est choisi parmi le méthanol, l'éthanol, le *n*-propanol, l'*iso*-propanol, le *n*-butanol, le *sec*-butanol, le *tertio-*butanol*.* De préférence, l'alcool est le méthanol, l'éthanol, le *n*-propanol et le *n*-butanol, de préférence le méthanol.

L'alcool et le sulfure d'hydrogène peuvent être introduits séparément dans le réacteur. Il est également possible de réaliser au préalable un mélange de ces réactifs.

Préalablement à l'étape a), on peut préparer un flux gazeux des réactifs de sulfure d'hydrogène et d'alcool comme suit. De l'alcool liquide est injecté dans du sulfure d'hydrogène gazeux. Cette injection permet de vaporiser partiellement ou entièrement l'alcool. Le mélange de sulfure d'hydrogène et d'alcool peut ensuite être entièrement vaporisé, si nécessaire de façon à obtenir un flux totalement gazeux.

Ainsi, un flux gazeux du mélange de sulfure d'hydrogène et d'alcool, de préférence préparé tel que ci-dessus, est introduit dans un réacteur. Il est également possible d'introduire dans le réacteur l'alcool et le sulfure d'hydrogène, chacun sous forme gazeuse.

Ledit réacteur peut être isotherme ou adiabatique, à plaques, multitubulaire ou à lit fixe. On choisit de préférence un réacteur adiabatique.

La température de réaction peut être comprise entre 200°C et 500°C, de préférence entre 200°C et 400°C. De préférence, la température de réaction est comprise entre 200°C et 360°C. Au-dessus de cette température, le catalyseur peut être physiquement endommagé (par frittage et cokage notamment).

La pression peut être comprise entre 1 et 40 bars absolus.

Le rapport molaire sulfure d'hydrogène/alcool peut être compris entre 0,1 et 100, de préférence entre 1 et 50, encore plus préférentiellement entre 1 et 20. Le sulfure d'hydrogène est de préférence en excès par rapport à l'alcool.

Le réacteur peut contenir un catalyseur pour la réaction de formation de l'alkylmercaptan, de préférence en phase gaz. Parmi les catalyseurs pouvant être utilisés, on peut citer :
- les catalyseurs à base d'alumine ;
- le dioxyde de thorium ThO₂, de préférence déposé sur un support silicaté ;
- les catalyseurs à base de sulfure de cadmium, de préférence sur un support alumine ;
- les catalyseurs à base des oxydes suivants : MgO, ZrO₂, TiO₂ rutile (R) et anatase (A), CeO₂, et γ-Al₂O₃ ;
- les catalyseurs à base d'oxydes de métaux, de préférence dopés par des métaux alcalins (Li, Na, K, Rb, Cs) et éventuellement supportés sur SiO₂, Al₂O₃ ou Nb₂O₅ ;
- les catalyseurs à base de carbonates de métaux alcalins ;
- les catalyseurs à base de sels de métaux alcalins avec certains acides de métaux de transitions (Cr, Mo, W, Ni), imprégnés sur l'alumine gamma ou autres oxydes de métaux ;
- le tungstate de potassium sur alumine K₂WO₄/Al₂O₃.

De préférence, les catalyseurs sont les oxydes d'alcalins imprégnés sur alumine, et plus préférentiellement encore, les oxydes de sodium ou de potassium sur alumine de type gamma.

On obtient ainsi un flux (M) comprenant de l'alkylmercaptan, de l'eau, éventuellement du sulfure d'hydrogène n'ayant pas réagi et des sous-produits soufrés.

### Etape supplémentaire de condensation

Le procédé selon l'invention peut comprendre au moins une étape de condensation du flux (M).

Le flux (M) issu de l'étape a) peut être condensé à l'aide de toute technique classique, de préférence à l'aide d'un ou plusieurs condenseur(s) ou économiseur(s). De préférence, le flux (M) est condensé à une température comprise entre 20°C et 70°C, par exemple entre 30°C et 60°C.

### Etape b) - Purification

Le procédé selon l'invention comprend au moins une étape de purification du flux (M).

De préférence, lors de l'étape b), ladite au moins une étape de purification correspond à au moins une étape de séparation de phases, de préférence par décantation, et/ou à au moins une étape de distillation. L'étape b) peut notamment correspondre à une ou plusieurs étape(s) de séparation de phases, par exemple une ou deux étapes de décantation, et/ou une ou plusieurs étape(s) de distillation, par exemple une ou deux étapes de distillation.

De préférence, l'étape b) permet par une ou plusieurs étape(s) de purification d'éliminer du flux (M) l'eau, le sulfure d'hydrogène n'ayant pas réagi et/ou les sous-produits soufrés éventuellement présents dans le flux (M). En particulier, suite à l'étape b), on obtient un flux enrichi en alkylmercaptan.

Préférentiellement, l'étape b) comprend au moins une étape de séparation de l'H₂S en particulier par distillation. De préférence, l'étape b) comprend au moins une étape de décantation et au moins une étape de distillation, ces deux étapes permettant de séparer l'H₂S du flux (M). Le(s)dite(s) étape(s) de décantation et/ou de distillation peuvent être effectuées selon les conditions décrites ci-dessous pour les étapes b1) et b2). L'étape de purification b) peut être effectuée par toute technique classique, et en particulier selon l'une des étapes b1) et/ou b2), de préférence selon les étapes successives b1) à b4), telles que décrites ci-dessous.

### Etape b1 - Séparation

Lors de l'étape b1) de séparation, de préférence par décantation, on obtient :
- un flux gazeux (M1) comprenant du sulfure d'hydrogène n'ayant pas réagi ; et
- un flux organique (M2) comprenant de l'alkylmercaptan, éventuellement de l'eau, éventuellement du sulfure d'hydrogène n'ayant pas réagi et éventuellement des sous-produits soufrés, et
- un flux aqueux (M3).

De préférence, le flux (M) est séparé à une température comprise entre 20°C et 70°C, de préférence entre 30°C et 60°C. La pression peut être comprise entre 1 et 40 bars absolus.

Le flux (M2) obtenu peut notamment être à l'état gazeux ou à l'état liquide. Lorsque le flux (M2) est à l'état gazeux, les flux (M1) et (M2) peuvent être combinés.

En particulier, le flux aqueux (M3), de préférence à l'état liquide, comprend au moins 50%, de préférence au moins 70%, plus préférentiellement au moins 90% en poids d'eau, par rapport au poids total de l'eau présente dans le flux (M). Le flux aqueux (M3) peut ensuite être envoyé vers un dégazeur. Le flux aqueux dégazé peut ensuite être envoyé au traitement des eaux usées.

Le flux gazeux (M1) peut être recyclé à l'alimentation du réacteur de l'étape a). Dans ce cas, une purge de ce flux (M1) peut être réalisée de façon à éviter l'accumulation d'inertes et/ou d'impuretés dans cette boucle de recyclage. On peut citer par exemple en tant qu'inertes et/ou impuretés : des alcanes gazeux, CO, CO₂, H₂ et N₂. Le flux gazeux résultant de cette purge est appelé E1. Lorsque les flux (M1) et (M2) sont combinés, le même type de purge peut être effectué de façon à obtenir un flux gazeux appelé E2.

Selon un mode de réalisation, les flux gazeux E1 ou E2 sont envoyés à l'incinération.

Selon un autre mode de réalisation, les flux gazeux E1 ou E2 peuvent être envoyés dans une colonne d'absorption à l'alcool, l'alcool étant celui choisi en tant que réactif, afin de récupérer les composés soufrés, tels que le sulfure d'hydrogène et/ou l'alkylmercaptan, qu'ils comprennent par une absorption gaz-liquide(alcool).

### Etape b2 - Elimination du sulfure d'hydrogène par distillation

Le flux (M) ou (M2) peut subir une distillation de façon à obtenir :
- un flux (M4) comprenant du sulfure d'hydrogène, de préférence en tête de colonne ; et
- un flux (M5) comprenant de l'alkylmercaptan, éventuellement de l'eau et éventuellement des sous-produits soufrés, de préférence en fond de colonne.

Lors de la distillation, la pression peut être comprise entre 0,05 et 40 bars absolus, de préférence entre 1 et 25 bars absolus et/ou la température peut être comprise entre -60°C et +60°C, de préférence entre 10 et 50°C, en tête de colonne ; et entre +20°C et +200°C, de préférence entre 20°C et 100°C, en fond de colonne.

Le flux (M4) comprenant le sulfure d'hydrogène peut être récupéré en tête de colonne, et éventuellement recyclé vers l'alimentation du réacteur de l'étape a).

En particulier, ladite distillation de l'étape b2) permet d'éliminer le sulfure d'hydrogène du flux (M) ou du flux (M2) (il est possible que des traces de sulfure d'hydrogène soient encore présentes dans le flux (M5)).

### Etape b3 - Eventuelle élimination des sous-produits soufrés par distillation :

On peut effectuer une distillation du flux (M2) ou du flux (M5) de façon à obtenir :
- un flux (M6) comprenant de l'alkylmercaptan et éventuellement de l'eau, de préférence en tête de colonne ; et
- un flux (M7) pouvant comprendre les sous-produits soufrés, de préférence en fond de colonne.

Lors de la distillation, la pression peut être comprise entre 1 et 40 bars absolus et/ou la température peut être comprise entre +20°C et + 100°C en tête de colonne, et entre +40°C et +200°C en fond de colonne.

En particulier, ladite distillation de l'étape b3) permet d'éliminer, lorsqu'ils sont présents, les sous-produits soufrés restant dans le flux (M2) ou (M5) (il est possible que des traces des sous-produits soufrés soient encore présentes dans le flux (M6)).

### Etape b4 - Eventuelle séparation de l'alkylmercaptan et des traces d'eau

Le flux (M2) ou le flux (M5) ou le flux (M6) peut subir une étape de purification supplémentaire visant à éliminer l'eau restante. Préalablement à cette étape b4), le flux (M2) ou le flux (M5) ou le flux (M6) peut être refroidi à une température aussi basse que possible, pour maximiser l'élimination de l'eau. De préférence, le flux (M2) ou le flux (M5) ou le flux (M6) est refroidi à une température comprise entre 20°C et 70°C, par exemple entre 30°C et 60°C.

Ce refroidissement permet de maximiser la séparation de l'eau potentiellement encore présente dans le flux, lors de l'étape b4). Lorsque l'alkylmercaptan est le méthylmercaptan, une température strictement supérieure à 16°C est maintenue pour éviter la formation d'hydrates solides de méthylmercaptan.

On peut ensuite effectuer une séparation de l'alkylmercaptan et de l'eau restante, de préférence par décantation, de façon à obtenir :
- un flux (M8) comprenant de l'alkylmercaptan, de préférence à l'état liquide ;
- un flux (M9) comprenant de l'eau, de préférence à l'état liquide.

En particulier, lors de l'étape b4), le flux (M9) comprend au moins 50% en poids, de préférence au moins 70%, plus préférentiellement au moins 90% en poids d'eau, par rapport au poids total de l'eau présente dans le flux (N).

Lors de l'étape de séparation b4), il est possible de récupérer la phase gazeuse ainsi séparée des flux (M8) et (M9), qui sont tous deux à l'état liquide. Ce flux gazeux est appelé E3.

Selon un mode de réalisation, le flux gazeux E3 est incinéré.

Selon un autre mode de réalisation, le flux gazeux E3 peut être envoyé dans une colonne d'absorption à l'alcool correspondant à l'alcool utilisé en tant que réactif, afin de récupérer les composés soufrés tels que le sulfure d'hydrogène et/ou l'alkylmercaptan, qu'ils comprennent par une extraction gaz-liquide.

### Etape supplémentaire de séchage du flux

Le flux (M2) ou le flux (M5) ou le flux (M6) ou le flux (M8) obtenu peut ensuite être séché.

Le séchage peut se faire sur tamis moléculaire, sur MgSO₄, par de l'H₂SO₄, sur CaCl₂ ou bien par distillation azéotropique, cette dernière n'est possible que, lorsque l'alcool utilisé en tant que réactif est le méthanol.

Le flux récupéré à l'issue de l'étape b) est noté (N).

### Etape c) - Récupération de l'alkylmercaptan

Le procédé selon l'invention comprend ensuite une étape de récupération de l'alkylmercaptan. Une partie du flux (N), notée (N1) est récupérée pour éventuellement être engagée dans un autre procédé. La seconde partie du flux (N), notée (N2) est, quant à elle, engagée dans l'étape suivante du procédé selon l'invention : l'étape d).

### Etape d) - Oxydation

Lors de l'étape d), on fait réagir une partie de l'alkylmercaptan (N2) obtenu à l'issu de l'étape c), par oxydation avec du soufre pour former un flux (O) comprenant du dialkyldisulfure, du sulfure d'hydrogène, éventuellement de l'alkylmercaptan n'ayant pas réagi, et éventuellement des dialkylpolysulfures.

Cette étape est par exemple décrite dans la demande de brevet EP 0 976 726. Par exemple, l'étape d) peut être conduite à chaud et sous pression, par exemple entre 20 et 200°C, de préférence entre 20 et 100°C, et la pression comprise entre 2 et 30 bars absolus, de préférence entre 2 et 15 bars absolus, typiquement par exemple à environ 70°C, sous environ 6 bars dans le cas de l'oxydation par le soufre du méthylmercaptan.

La réaction d'oxydation d) est réalisée dans un réacteur, qui peut contenir un catalyseur. De préférence, un catalyseur basique est utilisé. Ce catalyseur basique peut être homogène, biphasique ou hétérogène (solide). Quand le catalyseur est homogène, c'est-à-dire soluble dans le mercaptan, les amines, amidines, guanidines sont préférées. Quand le catalyseur basique forme une phase aqueuse biphasique, toutes bases hydrosolubles, telles que la soude, potasse, hydroxydes alcalins, alcalino-terreux, ammonium sont préférées. Quand la base envisagée est un solide, tout solide présentant un caractère basique est envisageable, tels que MgO, CaO, alumine ou tout autres supports (silice, zircones, oxydes de titane, hydrotalcites, hydroxyapathites...) dopés avec des oxydes d'alcalins, d'alcalino-terreux ou non, des zéolithes dopées ou non. De préférence, les catalyseurs basiques hétérogènes sont des résines échangeuses d'ions basiques, de manière encore préférée, le catalyseur hétérogène est la résine Amberlyst^{®} A21 commercialisée par la société Dupont.

Le rapport molaire alkylmercaptan/soufre de l'étape d'oxydation d) peut être compris entre 0,1 et 100, de préférence entre 1 et 50, plus préférentiellement encore entre 1 et 20.

Cette étape d'oxydation peut permettre de former un flux gazeux (012) comprenant du sulfure d'hydrogène et éventuellement de l'alkylmercaptan n'ayant pas réagi et un flux liquide (O11) comprenant le dialkyldisulfure, et éventuellement des dialkylpolysulfures résiduels.

### Etape supplémentaire de dégazage

Le flux (O) ou bien le flux liquide (O11) peut ensuite être traité dans un dégazeur de manière à éliminer du flux liquide les gaz résiduels, tels que le sulfure d'hydrogène ou l'alkylmercaptan éventuellement présent formant le flux (O22). Le flux liquide dégazé est nommé (021).

### Etape supplémentaire de rétrogradation des polysulfures

Le flux liquide (021) issu de l'étape précédente de dégazage supplémentaire ou bien le flux (O11) issu de l'étape d'oxydation peut subir une étape de rétrogradation des polysulfures de haut rang en soufre en polysulfures de plus bas rang, et idéalement en disulfures de manière à convertir les polysulfures résiduels en dialkyldisulfures. Le réacteur utilisé pour cette étape de rétrogradation est appelé finisseur. Il comporte une entrée d'alkylmercaptan introduit en excès de manière à augmenter la conversion de la réaction. Cette étape de finition peut permettre de former un flux gazeux (O32) comprenant du sulfure d'hydrogène et éventuellement de l'alkylmercaptan n'ayant pas réagi et un flux liquide (031) comprenant le dialkyldisulfure.

### Etape supplémentaire de dégazage

Le flux liquide (031) peut subir une étape supplémentaire de dégazage. Le flux liquide (031) peut être traité dans un dégazeur de manière à éliminer les gaz résiduels, tels que le sulfure d'hydrogène et éventuellement l'alkylmercaptan n'ayant pas réagi, formant le flux (O42). Le flux liquide dégazé est nommé (041).

### Etape e) - Purification

Le procédé selon l'invention comporte au moins une étape de purification du flux liquide issu de l'étape d'oxydation d). Ce flux liquide peut être le flux (O) issu directement de la réaction d'oxydation d) ou bien les flux (O11), (021), (031) ou (041) selon la présence d'étapes supplémentaires de dégazage ou de rétrogradation. Cette étape permet de séparer d'une part le dialkyldisulfure enrichi et d'autre part le sulfure d'hydrogène et éventuellement l'alkylmercaptan n'ayant pas réagi à l'étape d). Une telle étape peut notamment permettre de séparer :
- le dialkyldisulfure enrichi,
- le sulfure d'hydrogène avec éventuellement l'alkylmercaptan n'ayant pas réagi, et
- des impuretés telles que les produits lourds, les composés volatiles, les hydroalkyldisulfures ou les mercaptoalkylalkylsulfures.

Cette étape de purification e) peut comporter une ou plusieurs étape(s) de distillation permettant d'isoler le dialkyldisulfure. En particulier, ladite étape de purification e) peut comporter une ou plusieurs étape(s) de distillation, et éventuellement une ou plusieurs étape(s) de catalyse basique. En particulier, ladite étape de purification correspond à l'étape e1) ou e6) telles que décrites ci-dessous.

Selon un premier mode de réalisation, l'étape de purification e) peut être effectuée par toute technique classique et en particulier selon l'une ou les étape(s) successive(s) e1) à e4) telles que décrites ci-dessous.

### Etape e1) - Elimination du H₂S formé

Lors de l'étape e1) de purification, de préférence par distillation, on obtient :
- un flux gazeux (P12) comprenant du sulfure d'hydrogène et éventuellement de l'alkylmercaptan n'ayant pas réagi et éventuellement des impuretés volatiles ; et
- un flux liquide (P11) comprenant majoritairement du dialkyldisulfure.

Lors de la distillation, la pression peut être comprise entre 0.05 et 15 bars absolus, de préférence entre 1 et 10 bars absolus. La température en pied de colonne peut être comprise entre 50 et 300°C, de préférence entre 50 et 200°C. En tête de colonne, la température peut être comprise entre 30 et 200°C, de préférence entre 30 et 120°C.

### Etape e2) - Elimination des produits lourds

On peut effectuer ensuite une seconde distillation du flux issu de l'étape d) ou du flux (P11) de façon à obtenir :
- un flux (P22) constituant la tête de colonne et comprenant majoritairement du dialkyldisulfure et des traces résiduelles d'impuretés volatiles ; et
- un flux (P21) constituant le fond de colonne et comprenant un mélange d'impuretés lourdes.

Le flux des impuretés lourdes de distillation (P21) peut être recyclé vers l'étape de synthèse du dialkyldisulfure, notamment à l'étape d) ou e1), telles que définies ci-dessus. Il est possible d'équiper la conduite de recyclage d'une purge de manière à éviter l'accumulation des impuretés dans le procédé.

### Etape e3) - Elimination des hydroalkyldisulfures par réaction basique

On peut faire réagir le flux issu de l'étape d) ou le flux (P11) et/ou encore le flux (P22) dans un réacteur comportant un catalyseur basique de manière à convertir les hydroalkyldisulfures en trisulfure de dialkyle selon la réaction suivante :

RSSH + RSSR → RSH + RSSSR

Le catalyseur basique peut être de tout type connu de l'homme du métier. Ledit catalyseur basique est de préférence hétérogène par rapport au milieu réactionnel, de manière à faciliter sa séparation ultérieure. Ainsi, le catalyseur basique peut par exemple être choisi parmi les résines échangeuses d'anions, telle que l'Amberlyst^{®} A21 de Dupont, les catalyseurs basiques sous forme amine libre, les alumines dopées à l'oxyde de sodium et/ou à l'oxyde de potassium, l'oxyde de magnésium (MgO) et les zéolites basiques. Il est également possible d'utiliser les catalyseurs listés ci-dessus pour la réaction d'oxydation d). De préférence, le catalyseur basique est une résine échangeuse d'anions.

### Etape e4) - Elimination des traces de composés volatils

On peut effectuer enfin une troisième distillation du flux sortant de l'étape e3) de façon à obtenir :
- un flux (P31) en tête de colonne comprenant les traces d'alkylmercaptan, éventuellement formées à l'étape e3), et
- un flux (P32) constituant le fond de colonne et comprenant le disulfure de dialkyle.

Selon un second mode de réalisation, l'étape de purification e) peut être effectuée selon les étapes e5) et e6) telles que décrites ci-dessous.

### Etape e5) - Elimination des impuretés indésirables par réaction basique

Le flux liquide (O) issu directement de la réaction d'oxydation d) ou bien les flux (O11), (021), (031) ou (041) selon la présence d'étapes supplémentaires de dégazage ou de rétrogradation peuvent subir une réaction de catalyse basique. Ainsi, ces flux peuvent entrer dans un réacteur comportant un catalyseur basique de manière à éliminer les impuretés indésirables Le catalyseur utilisé peut être celui divulgué pour l'étape e3) définie ci-dessus.

### Etape e6) - Elimination des traces de composés volatils et des impuretés lourdes

On peut effectuer ensuite une distillation du flux sortant de l'étape e5) de façon à obtenir :
- un flux en tête de colonne comprenant du sulfure d'hydrogène et les traces d'alkylmercaptan,
- un flux éliminé en position latérale comportant le dialkyldisulfure, et
- un flux constituant le fond de colonne et comprenant le mélange d'impuretés lourdes.

La colonne à distillation utilisée pour réaliser cette étape peut être une colonne à soutirage latérale ou une colonne à paroi.

Si une colonne à paroi est utilisée, alors les conditions de la colonne peuvent être les suivantes. La température de la tête de colonne peut être comprise entre 0°C et 150°C, de préférence entre 10°C et 100°C. La température de milieu de colonne peut être comprise entre 30°C et 200°C, de préférence entre 50°C et 150°C. La température de pied de colonne peut être comprise entre 50°C et 250°C, de préférence entre 80°C et 180°C. La pression à l'intérieur de la colonne peut être comprise entre 0.05 bar et 30 bars absolus, de préférence entre 0.1 et 5 bars absolus. Le taux de reflux défini comme étant le rapport massique entre le liquide réinjecté en tête de colonne sur le distillat contenant les impuretés légères en tête de colonne, est compris entre 0 (pas de reflux) et 100, de préférence entre 0 et 10.

Selon un troisième mode de réalisation, il est possible d'incorporer une colonne de distillation préalablement aux étapes e5) et e6), afin d'éliminer les impuretés volatiles avant l'étape de catalyse basique.

### Etape de recyclage f)

L'étape f) de recyclage conduit le flux à recycler avant l'étape b) de purification, qui élimine le sulfure d'hydrogène du flux (M) à purifier, de préférence par distillation.

En effet, le sulfure d'hydrogène, et éventuellement l'alkylmercaptan récupéré(s) au cours de ces étapes d) et/ou e), et éventuellement des étapes supplémentaires, est recyclé vers le flux (M) issu de l'étape a), c'est-à-dire est injecté dans le flux (M) de manière à subir l'étape b) de purification, qui élimine le sulfure d'hydrogène du flux (M) à purifier, de préférence par distillation. De préférence, le flux recyclé est ré-injecté dans le milieu avant l'étape b1) et/ou l'étape b2). Ainsi, les flux (012), (O22), (O32), (O42), (P12) et (P31) peuvent être rassemblés en un seul flux et réinjectés vers le flux (M).

Une partie ou bien la totalité du flux peut être réinjecté dans le flux (M). Lorsqu'une partie du flux est réinjectée, la conduite de recyclage contient une purge, de manière à réguler les proportions du flux recyclé. De préférence, la totalité du flux de sulfure d'hydrogène, et éventuellement l'alkylmercaptan n'ayant pas réagi à l'étape d), est recyclée vers le flux (M) issu de l'étape a).

### Etape de récupération g)

Le dialkyldisulfure est enfin récupéré.

La figure 1 représente un mode de réalisation des étapes a) à g) du procédé selon l'invention.

L'étape a) de réaction est réalisée dans un réacteur A à partir d'alcool et de sulfure d'hydrogène.

Le flux d'alcool rentre par la conduite 1 dans le réacteur A. Le flux de sulfure d'hydrogène rentre par la conduite 2 dans le réacteur A. Le flux M sortant du réacteur A par la conduite 3 comprend de l'alkylmercaptan, de l'eau, du sulfure d'hydrogène n'ayant pas réagi et éventuellement des sous-produits soufrés.

L'étape b) de purification est réalisée dans un dispositif B, tel qu'un séparateur. Le flux de sulfure d'hydrogène est séparé et éliminé par une conduite 4, l'eau est éliminée par une conduite 6 et le flux N comprenant l'alkylmercaptan, et éventuellement des sous-produits soufrés sort du dispositif B via la conduite 5.

La conduite 4 est reliée à la conduite 2 amenant le sulfure d'hydrogène au réacteur A.

La conduite 5 est divisée en une conduite 7 et une conduite 8. La conduite 8 permet une récupération d'alkylmercaptan (étape c) du procédé) et la conduite 7 amène le reste du flux N au réacteur D.

L'étape d) d'oxydation est réalisée dans un réacteur D. Le soufre est introduit dans le réacteur D par la conduite 9. Le flux O sortant du réacteur D par la conduite 10 comprend du dialkyldisulfure, du sulfure d'hydrogène, de l'alkylmercaptan n'ayant pas réagi et éventuellement des sous-produits soufrés.

L'étape e) de purification est réalisée dans un dispositif E, tel qu'une colonne de distillation. Le flux de sulfure d'hydrogène et d'alkylmercaptan n'ayant pas réagi est éliminé en tête de colonne par une conduite 11, le pied de colonne est recyclé par une conduite 13 vers le réacteur D. Les conduites 11 et 13 peuvent comporter une purge. Le milieu de colonne comportant le dialkyldisulfure est récupéré par une conduite 12.

La conduite 11 recycle la tête de colonne comprenant le sulfure d'hydrogène et l'alkylmercaptan n'ayant pas réagi vers la conduite 3 menant le flux M vers le dispositif B de purification.

Les exemples, qui suivent permettent d'illustrer la présente invention, mais ne sont en aucun cas limitatifs.

### Exemples

### 1. Elimination des rejets soufrés

Deux unités de production de DMDS et de MeSH ont été comparées. L'une ne comporte pas l'étape f) de recyclage des flux après l'étape a). L'autre est une unité selon l'invention et comporte cette étape f) de recyclage. On produit 50 000 T/an de MeSH et 50 000 T/an de DMDS, soit pour chacun des 2 produits, une production de 151.5 T/jour (base 330 jours/an), ou 6.3 T/h (base 24h/j). Dans ces conditions, le flux 11 de la figure 1 contient 2.2 T/h d'H₂S et 2.3 T/h de MeSH.

| | Emission de SO₂ (T/h) | Perte en rendement MeSH sur l'ensemble des 2 unités (%) |
|---|---|---|
| Unité de production de 50000 tonnes /an de MeSH et de 50000 tonnes/an de DMDS sans recyclage (comparatif - incinération du flux 11) | 7.3 | 18.3 % |
| Unité de coproduction de 50000 tonnes /an de MeSH et de 50000 tonnes/an de DMDS avec recyclage (invention - recyclage du flux 11) | Négligeable- | Négligeable |

Le comparatif montre deux des avantages du procédé selon l'invention. Le premier avantage est d'éviter l'incinération de produits soufrés et la libération d'oxyde de soufre dans l'environnement, participant à la pollution atmosphérique. Le second est d'améliorer le rendement de la production de MeSH.

## Revendications

1. Procédé de co-production d'alkylmercaptan et de dialkyldisulfure comprenant les étapes successives suivantes :
a) réaction d'un alcool en C₁-C₄ en présence de sulfure d'hydrogène (H₂S) pour former un flux (M) comprenant un alkylmercaptan, de l'eau, et éventuellement du sulfure d'hydrogène n'ayant pas réagi,
b) purification du flux (M) pour obtenir un flux (N) enrichi en alkylmercaptan,
c) récupération d'une première partie du flux (N) comportant de l'alkylmercaptan purifié à l'étape b),
d) oxydation par du soufre de la seconde partie du flux (N) d'alkylmercaptan, pour former un flux (O) comprenant un dialkyldisulfure, du sulfure d'hydrogène, et éventuellement de l'alkylmercaptan n'ayant pas réagi,
e) purification du flux (O) pour séparer d'une part le dialkyldisulfure enrichi et d'autre part le sulfure d'hydrogène et éventuellement l'alkylmercaptan n'ayant pas réagi à l'étape d),
f) recyclage du sulfure d'hydrogène et éventuellement de l'alkylmercaptan isolés lors de l'étape e) vers le flux (M) issu de l'étape a),
g) récupération du dialkyldisulfure isolé à l'étape e).

2. Procédé selon la revendication 1, **caractérisé en ce que** l'étape f) de recyclage conduit le flux à recycler avant l'étape b) de purification, qui élimine le sulfure d'hydrogène du flux (M) à purifier, de préférence par distillation.

3. Procédé selon la revendication 1 ou 2, **caractérisé en ce que** la totalité du flux comportant le sulfure d'hydrogène, et éventuellement l'alkylmercaptan n'ayant pas réagi à l'étape d), est recyclé vers le flux (M) issu de l'étape a).

4. Procédé selon l'une quelconque des revendications précédentes, **caractérisé en ce que** l'alcool en C₁-C₄ est choisi parmi le méthanol, l'éthanol, le *n*-propanol, l'*iso*-propanol, le *n-*butanol, le *sec*-butanol, le *tertio-*butanol*,* de préférence l'alcool est le méthanol.

5. Procédé selon l'une quelconque des revendications précédentes, **caractérisé en ce que** le rapport molaire sulfure d'hydrogène/alcool de l'étape a) est compris entre 0,1 et 100, de préférence entre 1 et 50, plus préférentiellement encore entre 1 et 20.

6. Procédé selon l'une quelconque des revendications précédentes, **caractérisé en ce que** la réaction de l'étape a) est mise en œuvre en présence d'un catalyseur choisi parmi les catalyseurs à base d'alumine, le dioxyde de thorium ThO₂, de préférence déposé sur un support silicaté, les catalyseurs à base de sulfure de cadmium, de préférence sur un support alumine, les catalyseurs à base des oxydes suivants : MgO, ZrO₂, TiO₂ rutile (R) et anatase (A), CeO₂, et γ-Al₂O₃, les catalyseurs à base d'oxydes de métaux, de préférence dopés par des métaux alcalins et éventuellement supportés sur SiO₂, Al₂O₃ ou Nb₂O₅, les catalyseurs à base de carbonates de métaux alcalins, les catalyseurs à base de sels de métaux alcalins avec certains acides de métaux de transitions imprégnés sur l'alumine gamma, le tungstate de potassium sur alumine K₂WO₄/Al₂O₃.

7. Procédé selon l'une quelconque des revendications précédentes, **caractérisé en ce que** le rapport molaire alkylmercaptan/soufre de l'étape d'oxydation d) est compris entre 0,1 et 100, de préférence entre 1 et 50, plus préférentiellement encore entre 1 et 20.

8. Procédé selon l'une quelconque des revendications précédentes, **caractérisé en ce que** la température de réaction de l'étape d'oxydation d) est comprise entre 20° et 200°C et la pression est comprise entre 2 et 30 bars absolus.

9. Procédé selon l'une quelconque des revendications précédentes, **caractérisé en ce que** la réaction de l'étape d'oxydation d) est mise en œuvre en présence d'un catalyseur basique choisi parmi les catalyseurs homogènes, biphasiques ou hétérogènes, de préférence, le catalyseur est un catalyseur basique hétérogène, plus préférentiellement, le catalyseur est une résine échangeuse d'ions basiques.

10. Procédé selon l'une quelconque des revendications précédentes, **caractérisé en ce que** l'étape de purification e) comporte une ou plusieurs étape(s) de distillation, et éventuellement une ou plusieurs étape(s) de catalyse basique.

## Patentansprüche

1. Verfahren zur gleichzeitigen Herstellung von Alkylmercaptan und von Dialkyldisulfid, umfassend die folgenden aufeinander folgenden Schritte:
a) Umsetzen eines C₁-C₄-Alkohols in Gegenwart von Schwefelwasserstoff (H₂S), um einen Strom (M) zu bilden, der ein Alkylmercaptan, Wasser und gegebenenfalls nicht umgesetzten Schwefelwasserstoff umfasst,
b) Reinigen des Stroms (M), um einen an Alkylmercaptan angereicherten Strom (N) zu erhalten;
c) Rückgewinnen eines ersten Teils des im Schritt b) gereinigten, Alkylmercaptan enthaltenden Stroms (N),
d) Oxidieren des zweiten Teils des Stroms (N) von Alkylmercaptan mit Schwefel, um einen Strom (O) zu bilden, der ein Dialkyldisulfid, Schwefelwasserstoff und gegebenenfalls nicht umgesetztes Alkylmercaptan umfasst;
e) Reinigen des Stroms (O), um zum einen das angereicherte Dialkyldisulfid und zum anderen den Schwefelwasserstoff und gegebenenfalls das im Schritt d) nicht umgesetzte Alkylmercaptan zu trennen,
f) Rückführen des Schwefelwasserstoffes und gegebenenfalls des Alkylmercaptans, die bei dem Schritt e) abgetrennt wurden, zu dem aus dem Schritt a) hervorgegangenen Strom (M),
g) Rückgewinnen des im Schritt e) abgetrennten Dialkyldisulfids.

2. Verfahren nach Anspruch 1, **dadurch gekennzeichnet, dass** der Schritt f) des Rückführens den zurückzuführenden Strom vor den Schritt b) des Reinigens leitet, der den Schwefelwasserstoff aus dem zu reinigenden Strom (M) entfernt, vorzugsweise durch Destillation.

3. Verfahren nach Anspruch 1 oder 2, **dadurch gekennzeichnet, dass** der gesamte Strom, der den Schwefelwasserstoff und gegebenenfalls das im Schritt d) nicht umgesetzte Alkylmercaptan enthält, zu dem aus dem Schritt a) hervorgegangenen Strom (M) zurückgeführt wird.

4. Verfahren nach einem der vorhergehenden Ansprüche, **dadurch gekennzeichnet, dass** der C₁-C₄-Alkohol ausgewählt ist aus Methanol, Ethanol, n-Propanol, Isopropanol, n-Butanol, sec-Butanol, tert-Butanol, der Alkohol vorzugsweise Methanol ist.

5. Verfahren nach einem der vorhergehenden Ansprüche, **dadurch gekennzeichnet, dass** das molare Verhältnis Schwefelwasserstoff/Alkohol des Schritts a) zwischen 0,1 und 100, vorzugsweise zwischen 1 und 50, noch bevorzugter zwischen 1 und 20 liegt.

6. Verfahren nach einem der vorhergehenden Ansprüche, **dadurch gekennzeichnet, dass** die Reaktion des Schritts a) in Gegenwart eines Katalysators durchgeführt wird, der ausgewählt ist aus den Katalysatoren auf Basis von Aluminiumoxid, dem Thoriumdioxid ThO₂, vorzugsweise auf einem silikatischen Träger abgeschieden, den Katalysatoren auf Basis von Cadmiumsulfid, vorzugsweise auf einem Aluminiumoxidträger, den Katalysatoren auf Basis der folgenden Oxide: MgO, ZrO₂, TiO₂ Rutil (R) und Anatase (A), CeO₂ und γ-Al₂O₃, den Katalysatoren auf Basis von Metalloxiden, vorzugsweise mit Alkalimetallen dotiert und gegebenenfalls auf SiO₂, Al₂O₃ oder Nb₂O₅ geträgert, den Katalysatoren auf Basis von Alkalimetallcarbonaten, den Katalysatoren auf Basis von Alkalimetallsalzen mit bestimmten Säuren von Übergangsmetallen, die auf Gamma-Aluminiumoxid imprägniert sind, Kaliumwolframat auf Aluminiumoxid K₂WO₄/Al₂O₃.

7. Verfahren nach einem der vorhergehenden Ansprüche, **dadurch gekennzeichnet, dass** das molare Verhältnis Alkylmercaptan/Schwefel des Oxidationsschritts d) zwischen 0,1 und 100, vorzugsweise zwischen 1 und 50, noch bevorzugter zwischen 1 und 20 liegt.

8. Verfahren nach einem der vorhergehenden Ansprüche, **dadurch gekennzeichnet, dass** die Reaktionstemperatur des Oxidationsschritts d) zwischen 20° und 200 °C und der Druck zwischen 2 und 30 bar absolut liegt.

9. Verfahren nach einem der vorhergehenden Ansprüche, **dadurch gekennzeichnet, dass** die Reaktion des Oxidationsschritts d) in Gegenwart eines basischen Katalysators durchgeführt wird, der aus den homogenen, zweiphasigen oder heterogenen Katalysatoren ausgewählt ist, der Katalysator vorzugsweise ein heterogener basischer Katalysator ist, der Katalysator noch bevorzugter ein basisches Ionenaustauscherharz ist.

10. Verfahren nach einem der vorhergehenden Ansprüche, **dadurch gekennzeichnet, dass** der Reinigungsschritt e) einen oder mehrere Destillationsschritt(e) und gegebenenfalls einen oder mehrere Schritt(e) zur basischen Katalyse umfasst.

## Claims

1. Process for the co-production of alkyl mercaptan and of dialkyl disulfide, comprising the following successive steps:
a) reaction of a C₁-C₄ alcohol in the presence of hydrogen sulfide (H₂S) to form a stream (M) comprising an alkyl mercaptan, water, and possibly unreacted hydrogen sulfide,
b) purification of the stream (M) to obtain a stream (N) enriched in alkyl mercaptan,
c) recovery of a first portion of the stream (N) including the alkyl mercaptan purified in step b),
d) oxidation with sulfur of the second portion of the stream (N) of alkyl mercaptan, to form a stream (0) comprising a dialkyl disulfide, hydrogen sulfide, and possibly unreacted alkyl mercaptan,
e) purification of the stream (0) to separate, on the one hand, the enriched dialkyl disulfide and, on the other hand, the hydrogen sulfide and possibly the alkyl mercaptan that has not reacted in step d),
f) recycling of the hydrogen sulfide and possibly of the alkyl mercaptan isolated in step e) into the stream (M) obtained from step a),
g) recovery of the dialkyl disulfide isolated in step e).

2. Process according to Claim 1, **characterized in that** the recycling step f) leads the stream to be recycled before the purification step b), which removes the hydrogen sulfide from the stream (M) to be purified, preferably by distillation.

3. Process according to Claim 1 or 2, **characterized in that** all of the stream including the hydrogen sulfide, and possibly the alkyl mercaptan that has not reacted in step d), is recycled into the stream (M) obtained from step a).

4. Process according to any one of the preceding claims, **characterized in that** the C₁-C₄ alcohol is chosen from methanol, ethanol, n-propanol, isopropanol, n-butanol, sec-butanol and tert-butanol, and preferably the alcohol is methanol.

5. Process according to any one of the preceding claims, **characterized in that** the hydrogen sulfide/alcohol mole ratio of step a) is between 0.1 and 100, preferably between 1 and 50, even more preferentially between 1 and 20.

6. Process according to any one of the preceding claims, **characterized in that** the reaction of step a) is performed in the presence of a catalyst chosen from catalysts based on alumina, thorium dioxide ThO₂, preferably deposited on a silicate support, catalysts based on cadmium sulfide, preferably on an alumina support, catalysts based on the following oxides: MgO, ZrO₂, TiO₂ rutile (R) and anatase (A), CeO₂, and γ-Al₂O₃, catalysts based on metal oxides, preferably doped with alkali metals and optionally supported on SiO₂, Al₂O₃ or Nb₂O₅, catalysts based on alkali metal carbonates, catalysts based on alkali metal salts with certain transition metal acids impregnated on γ-alumina, potassium tungstate on alumina K₂WO₄/Al₂O₃.

7. Process according to any one of the preceding claims, **characterized in that** the alkyl mercaptan/sulfur mole ratio of the oxidation step d) is between 0.1 and 100, preferably between 1 and 50, even more preferentially between 1 and 20.

8. Process according to any one of the preceding claims, **characterized in that** the reaction temperature of the oxidation step d) is between 20°C and 200°C and the pressure is between 2 and 30 bar absolute.

9. Process according to any one of the preceding claims, **characterized in that** the reaction of the oxidation step d) is performed in the presence of a basic catalyst chosen from homogeneous, two-phase or heterogeneous catalysts; preferably, the catalyst is a heterogeneous basic catalyst; more preferentially, the catalyst is a basic ion-exchange resin.

10. Process according to any one of the preceding claims, **characterized in that** the purification step e) includes one or more distillation steps, and optionally one or more basic catalysis steps.
